# EUROPÄISCHE PATENTSCHRIFT

(11) **EP 1 265 666 B1**
(45) Veröffentlichungstag und Bekanntmachung des Hinweises auf die Patenterteilung: **27.02.2008**
(21) Anmeldenummer: 01909765.8
(22) Anmeldetag: 16.02.2001
(51) Int. Cl.: A61M 16/08, A61M 16/10, F16L 59/02, F16L 53/00

(54) **ATEMGASSCHLAUCHANORDNUNG ZUR ZUFUHR EINES ATEMGASES**
RESPIRATORY GAS HOSE SYSTEM FOR SUPPLYING A RESPIRATORY GAS
SYSTEME DE FLEXIBLE POUR ACHEMINER UN GAZ A RESPIRER

(30) Priorität: 18.02.2000 DE 10007506
(43) Veröffentlichungstag der Anmeldung: 18.12.2002
(73) Patentinhaber: MAP Medizin-Technologie GmbH, 82152 Martinsried (DE)
(72) Erfinder: KRESSIERER, Petra, 85435 Erding (DE); LANG, Bernd, 82166 Gräfelfing (DE); BIENER, Achim, 80636 München (DE); HEIDMANN, Dieter, 82335 Berg (DE)
(74) Vertreter: Heunemann, Dieter
(86) Internationale Anmeldenummer: PCT/EP2001/001751
(87) Internationale Veröffentlichungsnummer: WO 2001/060439

(56) Entgegenhaltungen:
- EP-A- 0 621 050
- WO-A-98/26826
- DE-U- 20 018 593
- US-A- 4 400 420
- US-A- 5 600 752

## Beschreibung

Die Erfindung betrifft eine Atemgasschlauchanordnung zur Zufuhr eines Atemgases zu einer Person.

Derartige Atemgasschlauchanordnungen finden insbesondere im Bereich der Schlafmedizin zur Behandlung schlafbezogener Atmungsstörungen Anwendung. So kann beispielsweise bereits durch einen vergleichsweise geringen Atemgasüberdruck eine pneumatische Schienung der oberen Atemwege erreicht werden, wodurch auf wirkungsvolle Weise der Gefahr von Atemwegsobstruktionen vorgebeugt werden kann.

Die Behandlung schlafbezogener Atmungsstörungen durch eine kontinuierliche Überdruckbeatmung wird allgemein als CPAP-Therapie bezeichnet. Bei den hierzu verwendeten bekannten CPAP-Geräten ist üblicherweise in einem schallisolierten Gehäuse eine Fördereinrichtung, insbesondere ein Gebläse angeordnet, dessen Förderdruck, ggf. über eine elektronische Regeleinrichtung auf die Atmungstätigkeit des Patienten abgestimmt wird. Das durch die Fördereinrichtung geförderte Atemgas, in der Regel Umgebungsluft, wird über eine flexible Atemgasschlauchanordnung dem Patienten zugeführt. Hierzu werden im Regelfall Atemmasken verwendet, die auf die Nase des Patienten aufgesetzt werden, ohne hierbei den Mund des Patienten abzudecken. Es hat sich gezeigt, daß die CPAP-Therapie eine effektive und physiologisch gut verträgliche Therapiemethode zur Behandlung schlafbezogener Atmungsstörungen darstellt. Die zur Verbindung der patientenseitig angeordneten Atemmaske mit dem CPAP-Getät vorgesehene flexible Schlauchleitung wird jedoch oft als störend empfunden.

Die DE-U-20018593 betrifft eine Schlauchanordnung für ein medizinisches Beatmungsgerät mit einem Atemschlauch, einem dem Atemschlauch anliegenden elektrischen Heizelement und einem den Atemschlauch umhüllenden Mantel, der in Längsrichtung geteilt mit einem Verschlussmittel versehen ist, wobei das Heizelement aus wenigstens einem in Längsrichtung des Atemschlauchs erstreckten, selbstregelnden Heizband besteht. Zur Stromversorgung ist die Anordnung mit einer Netzanschlussschnur 7 versehen.

Der Erfindung liegt die Aufgabe zugrunde, eine Vorrichtung zur Zufuhr eines Atemgases zu einem Patienten zu schaffen, die sich durch einen erhöhten Anwendungskomfort auszeichnet.

Diese Aufgabe wird erfindungsgemäß durch die in den unabhängigen Patentansprüches angegebenen Merkmale gelöst.

Dadurch wird es auf vorteilhafte Weise möglich, einen unmittelbaren Berührungskontakt der Schlauchleitung mit dem Patienten zu vermeiden und die Schlauchleitung in einer unter ästhetischen Gesichtspunkten vorteilhaften Weise zu verbergen, in besonders vorteilhafter Weise wird auch der Kondensatbildung in der Schlauchleitung vorgebeugt, so daß auch im Falle hoher absoluter. Feuchtigkeit des Atemgases sich im Inneren der flexiblen Schlauchleitung keine Kondensattropfen bilden können.

Gem. einer besonders bevorzugten Ausführungsform der Erfindung ist der flexible Mantelkörper aus einem weichen Textilmaterial - insbesondere Fleecematerial - gefertigt.

Der flexible Mantelkörper ist in vorteilhafter Weise derart dimensioniert, daß die flexible Schlauchleitung darin zwanglos aufgenommen ist Vorzugsweise beträgt der Innendurchmesser des flexiblen Mantelkörpers wenigstens das 1,3-Fache des Außendurchmessers der flexiblen Schlauchleitung. Durch das zwischen der Außenwandung der flexiblen Schlauchleitung und dem flexiblen Mantelkörper gebildete Luftpolster wird eine weitere Verbesserung des Anwendungskomforts sowie der Isolierwirkung erreicht

Es ist möglich, den flexiblen Mantelkörper aus einem mehrlagigen Material zu bilden, das erst im Außenbereich eine unter ästhetischen Gesichtspunkten ausgewählte, angenehme Außenlage aufweist.

Der flexible Mantelkörper weist gem. einer besonders bevorzugten Ausführungsform der Erfindung im Bereich seiner Enden eine Schließeinrichtung auf, durch welche der Endbereich des flexiblen Mantelkörpers verengt, beispielsweise eingeschnürt, und vergleichsweise festsitzend an der flexiblen Schlauchleitung fixiert werden kann. Als geeignete Fixiereinrichtung wird gem. einer besonders bevorzugten Ausführungsform der Erfindung eine Zurrschnur verwendet, die durch Ösen und/oder einen Hohlsaum hindurchgeführt ist und ein Zusammenschnüren des entsprechenden Endes des flexiblen Mantels ermöglicht. Alternativ hierzu oder auch in Kombination damit ist es möglich, im Endbereich des flexiblen Mantels eine Klettverschlußeinrichtung vorzusehen, über welche das Ende des flexiblen Mantelkörpers entsprechend verengt werden kann.

Die Wanddicke des flexiblen Mantelkörpers liegt vorzugsweise im Bereich von 1-7 mm. Das verwendete Material kann beispielsweise ein Vollmaterial (Vliesmaterial) oder auch ein mehrlagiges Material sein. In besonders vorteilhafter Weise ist eine flexible Textillage auf eine Polsterlage, beispielsweise aus Schaumstoffmaterial aufkaschiert.

Der flexible Mantelkörper ist vorzugsweise als schlauchartiger Körper ausgebildet und hierbei auf die flexible Schlauchleitung aufgeschoben.

Alternativ dazu ist es auch möglich, den flexiblen Mantelkörper aus einem Streifenmaterial zu bilden, das eine sich in dessen Längsrichtung erstreckende Verbindungseinrichtung aufweist. Diese Verbindungseinrichtung ist in vorteilhafter Weise durch ein Klettverschlußband gebildet. Alternativ hierzu sind auch Reißverschluß-Verbindungseinrichtungen oder auch Knopflochanordnungen sowie Druckknöpfe möglich.

In besonders vorteilhafter Weise ist es möglich, im Bereich des CPAP-Gerätes sowie vorzugsweise auch im Bereich der Atemmaske Verbindungsmittel vorzusehen, über welche der flexible Mantelkörper zuverlässig fixiert werden kann. In vorteilhafter Weise bestehen diese Fixiermittel aus einem Vlies- oder Kletthakenband, das im jeweiligen Endbereich (Maske oder CPAP-Gerät) am Schlauch fixiert vorzugsweise angeklebt oder geklemmt ist.

Es ist gemäß einer besonders bevorzugten Ausführungsform der Erfindung auch möglich, im Bereich der Enden des Mantelkörpers elastische Bündchen vorzusehen durch welche das Mantelmaterial des Mantelkörpers auf den Schlauch oder die Schlauch-Endstücke - gedrängt wird. Hierzu ist es beispielsweise möglich, ein Gummiband im Endbereich des Mantelkörpers vorzusehen. Das Gummiband ist hierbei vorzugsweise in einem Hohlsaum aufgenommen.

Es ist in vorteilhafter Weise möglich, im Bereich der Auflagezonen des Mantelkörpers auf dem Schlauch eine rutschfeste beispielsweise gummierte Lage vorzusehen.

Die Wärme- und Schallisolationswirkung kann in vorteilhafer Weise durch die Matedalbeschaffenheit und den Aufbau des Mantelkörpers bedarfsgerecht abgestimmt werden. Eine besonders hohe Wärmeisolationswirkung wird erreicht, indem mehrere Lagen Fleece-Material verarbeitet werden. Es ist möglich, die Lagen des Fleece-Materiales vorzugsweise in Verbindung mit Deck- und Futterlagen abzusteppen oder zu quilten.

Zumindest der Außenbereich des Mantelkörpers ist vorzugsweise mehrfarbig ausgebildet. Diese Mehrfarbigkeit wird vorzugsweise durch Vernähen mehrfarbiger Textilabschnitte erreicht, wobei die Vebindungsnähte hierbei gleichzeitig als Quiltnähte dienen.

Es ist möglich, in den Mantelkörper eine Band- oder Stab-artige Versteifungseinlage einzubinden, so daß bei einer schlauchartigen Ausgestaltung des Mantelkörpers eine Aufzieh-Hilfe erreicht wird.

Im Falle einer längsgeteilten Ausführungsform des Mantelkörpers ist es möglich, alternativ zu Klett- oder Knopf- insbesondere Druckknopf-Verbindungen hier einen Längsreißverschluß vorzusehen. Dieser Längsreißverschluß ist vorzugsweise derart überlappt ausgebildet, daß die Reißverschlußstruktur nach außen überdeckt ist. Es ist auch möglich, hier eine Magnetverschlußeinrichtung vorzusehen.

Gemäß der Erfindung ist in den Mantelkörper eine Heizeinrichtung integriert. Diese Heizeinrichtung kann durch eine elektrische Widerstandsheizung - gebildet sein. Gemäß einer besonders bevorzugten Ausführungsform umfaßt die. Heizeinrichtung eine Niedervoithelzfolie, wobei das Leistungsaufhahmeverhalten dieser Heizfolie derart gewählt ist, daß die Heizfolie eine vorgegebenen Heiztemperatur selbstregelnd nicht überschreitet Die Heizfolie kann in den Mantelkörper eingenäht sein, wobei vorzugsweise das Wärmeisolationsvermögen des Mantelkörpers außerhalb der Heizfolie größer ist als im Bereich zwischen Heizfolie und Schlauch. Alternativ hierzu ist es auch möglich, den Schlauch als beheizbaren Atemgasschlauch auszubilden.

Die Spannungsversorgung der Heizeinrichtung erfolgt vorzugsweise über eine Niedervoitleitung die mit einem Anschlußstecker versehen ist welcher in eine entsprechende Spannungsversorgungsbuchse eines Netzgeräts - oder vorzugsweise eine, an einem CPAP-Gerät, oder einem Luftbefeuchter vorgesehene Spannungsversorgungsdose einsteckbar ist. Durch einen derart beheizbar ausgebildeten Mantelkörper wird neben einer zuverlässigen Vermeidung von Kondensationserscheinungen auch Temperierung des Atemgases ermöglicht

Es ist möglich, im Bereich des Mantelkörpers Isolationsmittel zur Wärmestrahlungsabschirmung vorzusehen. Vorzugsweise ist hierbei auf einer an die Schlauchleitung angrenzenden Innenseite des Mantelkörpers eine metallisierte beispielsweise Aluminium, Silber- oder Gold-bedornpfte Superisolationsfolie vorgesehen.

Gemäß einer besonders bevorzugten Ausführungsform der Erfindung sind an dem Mantelkörper wenigstens eins - vorzugsweise mehrere Fixierstellen vorgesehen, über weiche der Mantelkörper - und damit die gesamte Schlauchanordnung aufgehängt oder definiert in ihrer räumlichen Bewegbarkeit festgelegt werden können. Diese Fixierstellen können beispielsweise durch Ösen ausgebildet sein. Vorzugsweise sind zwei bis vier derartige Ösen zueinander beabstandet entlang der Schlauchleitung angeordnet.

Gemäß einer besonders bevorzugten Ausführungsform der Erfindung ist der Mantelkörper derart ausgestaltet, daß dieser einen Behälterabschnitt bildet, in welchen der übrige Teil des Mantelkörpers hineingesteckt, werden kann. Dieser in den Mantelkörper integrierte Behälterabschnitt kann als flacher Beutel oder Taschenbereich ausgebildet sein der durch Aufnähen einer flexiblen Lage auf eine Außenfläche des Mantelkörpers gebildet ist. Es ist auch möglich, den überwiegenden Teil des Mantelkörpers in den Innenbereich eines seiner Längsendabschnitte hinein zu zwängen. Hierzu ist vorzugsweise ein Längsabschnitt des Mantelkörpers sackartig ausgebildet.

Eine besonders gewichtssparende Ausführungsform des Mantelkörpers wird dadurch erreicht, daß wenigstens eine der isolierenden Lagen als Luftpolster-Lage ausgebildet ist.

Der Mantelkörper ist gemäß einem besonderen Aspekt der vorliegenden Erfindung derart ausgebildet, daß dessen Biegeverhalten in Längsrichtung des Mantelkörpers betrachtet, variiert. So ist es beispielsweise möglich, im maskennahen Bereich des Mantelkörpers eine hohe Flexibilität vorzusehen, wogegen im gerätenahen Bereich der Mantelkörper steifer ausgebildet ist. Das Verformungsverhalten des Mantelkörpers kann hierbei durch Gelenk- und/oder Biegematerialeinlagen bestimmt werden.

Der Mantelkörper ist gemäß einer besonders bevorzugten Ausführungsform der Erfindung mit einem Knickschutz beispielsweise in Form von Spiral- oder schlangenlinienartig verlaufenden Einlagen versehen. Hierdurch wird es möglich, den in dem Mantelkörper aufgenommenen Atemgasschlauch dünnwandig - ggf. als Einweg-Leicht-Schlauch auszubilden.

Der Mantelkörper kann gemäß einer besonders bevorzugten Ausführungsform der Erfindung auch länger ausgebildet sein als die eigentliche Schlauchleitung. Hierdurch wird es möglich, durch den Mantelkörper auch Zusatzfunktionselemente abzudecken. So ist es in Verbindung mit dem Mantelkörper möglich, beispielsweise modular an die Schlauchleitung angekoppelte Elemente durch den Mantelkörper abzudecken und zu einer nach außen hin abgeschlossen erscheinenden Einheit zu kombinieren.

Es ist insbesondere möglich, einen rohr- oder schfauchförmig ausgebildeten Luftbefeuchter, ein Gasanalysemodul, einen Gasfluß-Sensor, eine Druckschleuse oder dgl. maskennah modulartig an die Schlauchleitung anzukoppeln und durch den Mantelkörper abzudecken und bedarfsweise zu isolieren. Durch den Mantelkörper sind hierbei in vorteilhafter Weise etwaige Zusatzleitungen elektrischer, optischer, oder auch pneumatischer Art abgedeckt.

Der Mantelkörper bildet hierbei ein flexibles vorzugsweise wärme- und schallisolierendes, die einzelnen Module übergreifendes, und hierbei zusammenfassendes, Gehäuseteil.

Der Mantelkörper kann mit mehreren Öffnungen versehen sein, durch weiche Leitungseinrichtungen beispielsweise einer Wasserfalle aus dem Mantelkörper herausgeführt werden können. Diese Öffnungen sind vorzugsweise über eine Deckeleinrichtung verschließbar.

Weitere Einzelheiten der Erfindung ergeben sich aus der nachfolgenden Beschreibung unter Bezugnahme auf die beigefügte Zeichnung. Es zeigen;
- **Fig. 1**: eine perspektivische Ansicht einer CPAP-Atemgasschlauchanorduung, mit einem aus einem textilen Weichmaterial gefertigten, Mantelkörper,
- **Fig. 2a**: eine vereinfachte Schnittansicht, durch einen Abschnitt eines mehrlagig ausgebildeten Mantelkörpers;
- **Fig. 2b**: eine vereinfachte Schnittansicht, durch einen Abschnitt eines einlagig ausgebildeten Mantelkörpers;
- **Fig. 2c**: eine vereinfachte Schnittansicht, durch einen Abschnitt eines zweilagig mit einer textilen Decklage ausgebildeten Mantelkörpers;
- **Fig. 3**: eine perspektivische Darstellung zur Erläuterung einer im Endbereich des flexiblen Mantelkörpers vorgesehenen Fixiereinrichtung;
- **Fig. 4**: eine Ausführungsform des flexiblen Mantelkörpers als längsgeteilter Körper einschließlich der sich entlang der entsprechenden Längskante ersteckenden, hier als Klettverschluß ausgebildeten Verbindungseinrichtung;
- **Fig.5**: eine perspektivische Skizze zur Erläuterung einer bevorzugten Ausführungsform eine erfindungsgemäßen Atemgasschlauchanordnung;
- **Fig.6**: eine Skizze eines Abschnittes des Mantelkörpers hier mit Ösenabschnitten zur Aufhängung des Mantelkörpers;
- **Fig.7**: eine Skizze zur Erläuterung eines in den Mantelkörper abschnittsweise einsetzbaren Gelenkeinsatzes;
- **Fig.8**: eine Skizze einer kartuschenartig ausgebildeten Gebläseeinrichtung (CPAP-Druckquelle) zum Einsatz in den Mantelkörper.

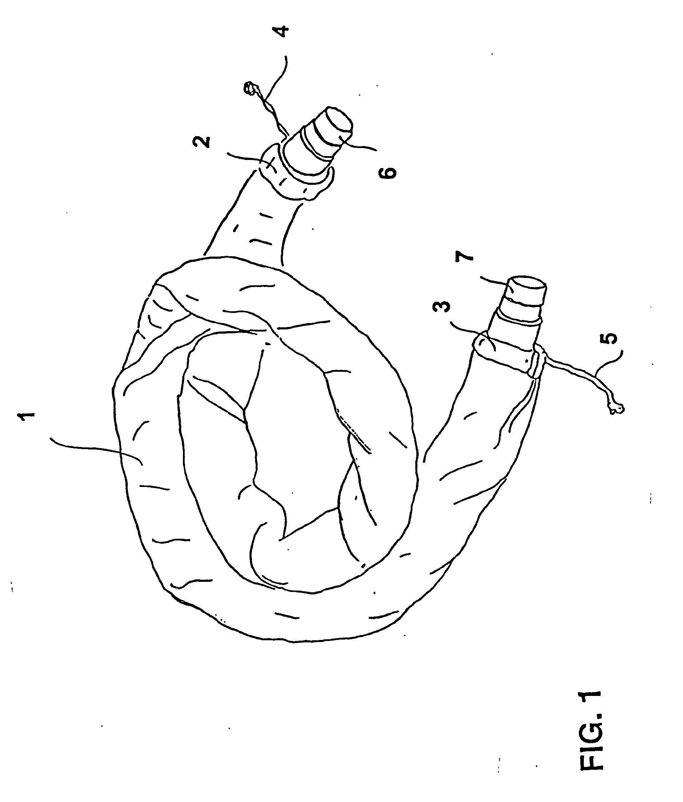

Die in Fig. 1 dargestellte Aterngasschlauchanordung umfaßt eine hier nicht sichtbare, aus einem spiralverstärkten Kunststoffmaterial gebildete flexible Schlauchleitung und einen aus einem Isolierenden Textilmaterial - hier Vilesmaterial - gebildeten isolierenden Mantelkörper 1. Der Mantelkörper 1 ist aus einem schmalen Stoffstreifen gebildet, der entlang einer Längsnaht (Overlocknaht) zu einem Schlauch vernäht ist. Im Endbereich des Mantelkörpers 1 ist bei der hier dargestellten Ausführungsform jeweils ein Hohlsaum 2, 3 ausgebildet, durch welchen jeweils eine Schnur 4. 5 hindurchgeführt ist Durch entsprechendes Ziehen der Schnüre 4, 5 können die Endbereiche des Mantelkörpers 1 zusammengezogen werden, wodurch auf vorteilhafte Weise der Mantelkörper 1 an der flexiblen Schlauchleitung fixiert ist Zum Anschluß der flexiblen Schlauchleitung an ein CPAP-Gerät, bzw. an eine Atemmaske sind im jeweiligen Schlauchendbereich Elastomerbuchsen 6, 7 vorgesehen, die elastisch auf eine entsprechend komplementäre Anschlußstruktur auf, bzw. in diese eingesteckt werden können.

Der Mantelkörper 1 ist derart dimensioniert, daß die flexible Schlauchleitung darin im wesentlichen zwanglos aufgenommen ist. Das hierbei zwischen der Innenfläche des Mantelkörpers 1 und der Außenfläche der flexiblen Schlauchleitung gebildete Luftpolster führt zu einer ohne Gewichtszunahme verbesserten Wärmeisolierung und verleiht der Atemgasschlauchanordnung zudem einen angenehmen Polstereffekt.

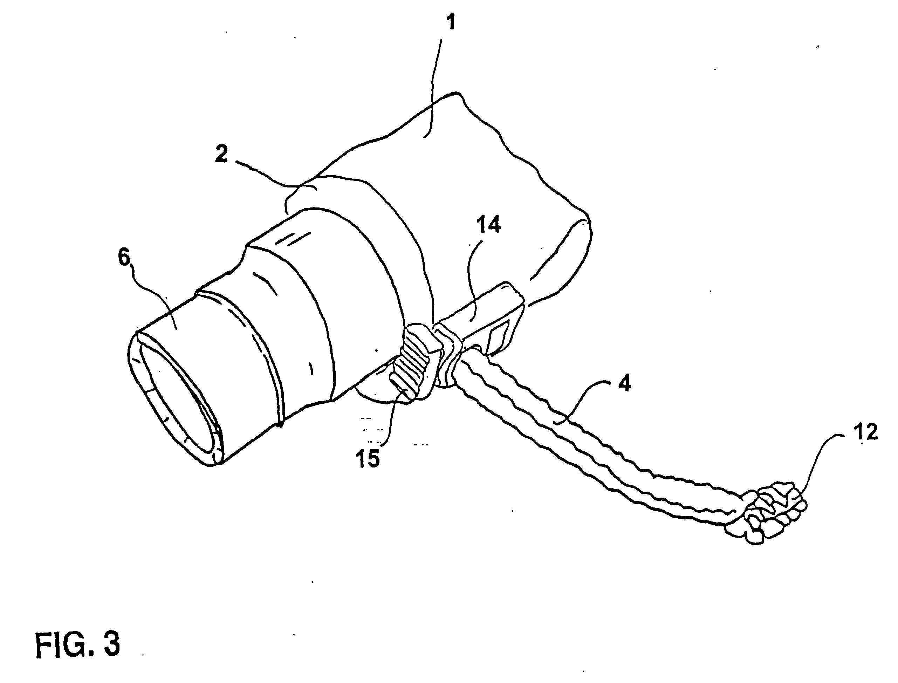

Zur Fixierung der Schnüre 4, 5 in einer entsprechenden Zurrposition sind - wie nachfolgend unter Bezugnahme auf Fig. 3 noch ausführlich erläutert werden wird, vorzugsweise Fixiermittel (hier nicht sichtbar) vorgesehen.

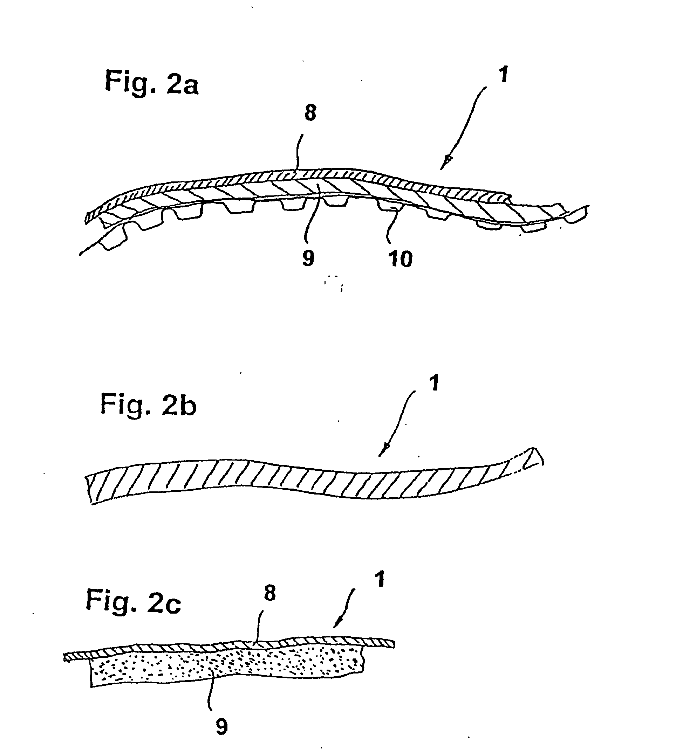

Der Mantelkörper 1 ist vorzugsweise, wie in Fig. 2a andeutungsweise dargestellt, mehrlagig ausgebildet. So kann beispielsweise als oberste, bzw. äußere Decklage eine unter ästhetischen Gesichtspunkten ausgewählte textile Decklage 8 auf einen polsternden und isolierenden Unterbau, der hier aus einer Schaumstofflage 9 und einer Noppenfolie 10 besteht, aufkaschiert werden. Die Verbindung zwischen den einzelnen Lagen kann durch entsprechende Verklebung, durch Nähte oder vorzugsweise durch Wärmekaschieren erfolgen. So ist es beispielsweise möglich, die isolierende Schaumstofflage 9 auf einem Schaumstoffmaterial zu bilden, dessen Deckfläche bei einer vorbestimmten Schmelztemperatur mit der äußeren Lage 8 und der Noppenfolie 10 verschweißt.

Alternativ zu dem beschriebenen mehrlagigen Aufbau ist es auch möglich, den Mantelkörper 1 aus einem Vollmaterial, vorzugsweise aus einem Vlies- oder Filzmaterial zu bilden, wie dies in Fig. 2b dargestellt ist. Vorzugsweise werden hierbei Materialien verwendet, die eine einfache Reinigung, vorzugsweise einen Kochwaschvorgang gestatten.

In Fig. 2c ist eine weitere Ausführungsform des Wandungsmaterials des flexiblen Mantelkörpers 1 dargestellt, das hier aus einer polsternden und wärmeisolierenden Schaumstofflage 9 und einer aus einem dekorativen Gewebematerial gebildeten Decklage 8 besteht. Die Verbindung zwischen der Decklage 8 und dem Schaumstoffmaterial 9 ist hier durch einen Flammkaschiervorgang erreicht.

In Fig. 3 ist eine bevorzugte Ausführungsform einer Fixiereinrichtung zur Fixierung des flexiblen Mantelkörpers 1 im Endbereich des flexiblen Atemgasschlauches dargestellt. Die Fixiereinrichtung umfaßt hier eine durch einen Hohlsaum 2 hindurchgeführte Schnur 4, die im Bereich ihrer herausgeführten Enden mit einem Knoten 12 versehen ist. Durch entsprechendes Ziehen an dem herausgeführten Abschnitt der Schnur 4, bzw. dem Knoten 12 kann das Ende des flexiblen Mantelkörpers 1 zusammengeschnürt werden.

Mittels einer Fixiereinrichtung 14 kann die Schnur 4 in der gespannten Stellung gehalten werden. Durch Betätigung einer hier als Druckknopf 15 ausgebildeten Löseeinrichtung kann die Schnur 4 gelockert und hierbei das zunächst eingeschnürte Ende des Mantelkörpers 1 geweitet werden. Eine besonders sichere Fixierung des Mantelkörpers 1 an der flexiblen Schlauchleitung kann dadurch erreicht werden, daß im Bereich des hier als elastomere Buchse 6 ausgebildeten Abschlußelementes eine Struktur ausgebildet ist, die mit dem entsprechenden Endabschnitt des Mantelkörpers 1 in Eingriff bringbar ist. Beispielsweise kann die elastomere Buchse 6 mit einer Umfangsnut oder einem Umfangswulst versehen sein, an welchem der entsprechend zusammengeschnürte textile Mantelkörper 1 fixierbar ist.

Alternativ zu der hier dargestellten Zurranordnung ist es auch möglich, das Ende des Mantelkörpers 1, beispielsweise über eine Klettverschlußeinrichtung oder Schnallen an der flexiblen Schlauchleitung (nicht dargestellt) zu fixieren.

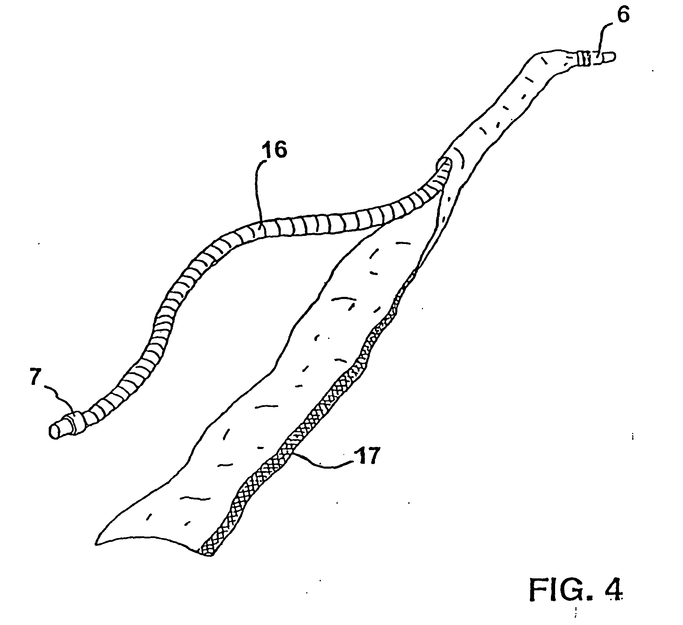

In Fig. 4 ist eine weitere Ausführungsform der Atemgasschlauchanordnung dargestellt, die hier eine flexible Schlauchleitung 16 aufweist, an deren Endbereich die elastomeren Buchsen 6, 7 vorgesehen sind. Abweichend von der vorangehend in Verbindung mit der in Fig. 1 beschriebenen Ausführungsform ist hier der flexible Mantelkörper 1 aus einem flexiblen Textilstreifen gebildet, der um die flexible Schlauchleitung 16 herumgeführt und entlang seiner Längskante mit einer Verbindungseinrichtung 17 versehen ist; so daß der um die Schlauchleitung 16 herumgeführte Materialstreifen ebenfalls schlauchartig die flexible Schlauchleitung 16 umgibt. In besonders vorteilhafter Weise ist hierzu im Bereich einer der Längskanten des Textilmaterialstreifens ein Klettverschlußband vorgesehen.

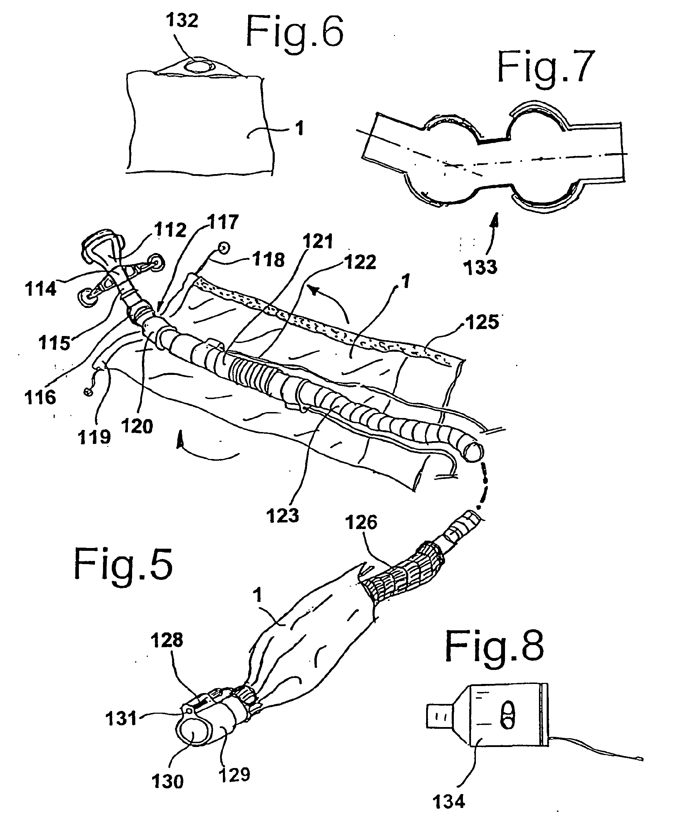

In Fig.5 ist eine weitere Ausführungsform der in Verbindung mit dem Mantelkörper 1 gebildeten Atemgasleitungseinrichtung einschließlich Atemmaske 112 dargestellt.

Die Atemmaske 112 ist über ein, in einen Stirnabstandshalter 114 integriertes Auswaschventil 115 an ein Gelenkstück 116 angeschlossen. Im Bereich des Gelenkstückes 116 ist eine Umfangsnut 117 ausgebildet, in weicher mittels eines Zurrbandes 118 ein vorderer Bundbereich 119 des Mantelkörpers 1 festlegbar ist.

An das Gelenkstück 116 schließt sich ein Atemgasstrom-Meßglied 120 an, das über eine hier nicht sichtbare Meßleitung mit einer Atemgasdruckquelle, beispielsweise einem CPAP-Gerät verbindbar ist.

Die Atemgasleitungseindchtung umfaßt weiterhin einen Atemgasbefeuchter 121, der über eine Befeuchtungsleitung 122 mit einer Wasserquelle verbindbar ist. Der Atemgasbefeuchter 121 ist als flexibles Rohrstück ausgebildet und zwischen Atemmaske 112 und dem überwiegenden Teil des Atemgasschlauches 23 angeordnet.

Im Bereich des Atemgasbefeuchters 121 ist der Mantelkörper 1 mit einer Superisolations-Folie versehen, so daß im Befeuchterbereich eine hohe Wärmeisloationswirkung erreicht wird. Hierdurch wird es möglich, die zur Verdunstung des Befeuchtungswassers erforderliche Wärme ausschließlich - oder zumindest überwiegend - aus der Atemluft zu beziehen.

Der Mantelkörper 1 fügt sich in Schließstellung schlauchartig über die aus mehreren Modulen zusammengesetzte Atemgasleitung und schließt diese nach außen ab. An dem Mantelkörper 1 ist bei dem gezeigten Ausführungsbeispiel ein Klettverschlußband 125, vorgesehen.

Die Atemgasleitung umfaßt weiterhin einen Schläuchschalldämpfer 126, der hier eine Länge von ca. 80 bis 120 cm aufweist und eine weitere Absorption etwaiger Geräusche einer Atemgasdruckquelle ermöglicht. Auch dieser Schalldämpfer 126 wird bei der hier gezeigten Ausführungsform noch von dem Mantelkörper 1 ummantelt.

In den Mantelkörper 1 ist eine Heizeinrichtung integriert. Die Spannungsversorgung dieser Heizeinrichtung erfolgt über Kontaktelemente 128, die in einen Anschlußstecker 129 integriert sind. Der Anschlußstecker 129 umfaßt einen Atemgasdurchgangsquerschnitt 130 sowie einen Druckmeßschlauchanschlußquerschnitt 131.

In Fig.6 ist ein Abschnitt des Mantelkörpers 1 dargestellt, der hier mit einer Öse 132 versehen ist, über welche der Mantelkörper 1 aufgehängt werden kann.

In Fig.7 ist ein Gelenkgliederelement 133 dargestellt, das koaxial zur Atemgasleitung in den Mantelkörper 1 einsetzbar ist. Das Gelenkgliederelement 133 umfaßt eine Vielzahl an Gelenkgliedern, die derart eng sitzend miteinander gekoppelt sind, daß die Atemgasleitungsanordnung in eine gewünschte Form biegbar ist.

In Fig.8 ist eine Gebläseeinrichtung 134 dargestellt, die hinsichtlich ihrer äußeren Abmessungen derart bemessen ist, daß diese ebenfalls noch in den Mantelkörper 1 einsetzbar ist. Hierdurch wird es möglich, eine schal- oder schlangenartig ausgebildete, modulartig auf den jeweiligen Anwender abgestimmte CPAP-Einrichtung zu schaffen.

## Patentansprüche

1. Flexible Atemgasschlauchanordnung zur Zufuhr eines Atemgases zu einer Person, aufweisend:
eine flexible Schlauchleitung (123);
einen flexiblen Mantelkörper (1);
eine Heizeinrichtung zum Erwärmen des durch die Schlauchleitung (123) geförderten Gases;
an den Enden der Schlauchleitung (123) vorgesehene Anschlusselemente, um die Schlauchleitung (123) mit einer Atemmaske (112) bzw. einer Gebläseeinrichtung zu verbinden; wobei
das Anschlusselement zur Verbindung mit einer Gebläseeinrichtung als Anschlussstecker ausgebildet ist, der einen Atemgasdurchgangsquerschnitt (130) sowie Kontaktelemente (128) aufweist, über die die Heizeinrichtung mit Spannung versorgt wird.

2. Atemgasschlauchanordnung gemäß Anspruch 1, wobei der isolierende Mantelkörper (1) lösbar mit der flexiblen Schlauchleitung (123) verbindbar ist.

3. Atemgasschlauchanordnung nach einem der Ansprüche 1 oder 2, wobei die Heizeinrichtung in den Mantelkörper (1) integriert ist.

4. Atemgasschlauchanordnung nach einem der Ansprüche 1 bis 3, wobei der Mantelkörper (1) mehrschichtig ausgebildet ist.

5. Atemgasschlauchanordnung nach Anspruch 1, wobei die Schlauchleitung (123) eine beheizbare Atemgasschlauchanordnung ist.

6. Atemgasschlauchanordnung gemäß Anspruch 1, wobei die Heizeinrichtung in die Schlauchleitung (123) integriert ist.

7. Atemgasschlauchanordnung nach einem der Ansprüche 1 bis 6, wobei der Anschlussstecker ferner einen Druckmessschlauchanschluss (131) aufweist.

8. Atemgasschlauchanordnung nach einem der Ansprüche 1 bis 7, ferner mit einem Atemgasbefeuchter (121).

9. Atemgasschlauchanordnung nach Anspruch 8, wobei der Atemgasbefeuchter (121) über eine Befeuchtungsleitung mit einer Wasserquelle verbindbar ist.

10. Atemgasschlauchanordnung gemäß Anspruch 8 oder 9, wobei der Atemgasbefeuchter (121) als flexibles Rohrstück ausgebildet ist, das zwischen der Atemmaske (112) und dem Atemgasschlauch (123) angeordnet ist.

11. Atemgasschlauchanordnung nach einem der Ansprüche 1 bis 10, wobei die flexible Schlauchleitung (123) aus einem spiral verstärkten Kunststoffmaterial gebildet ist.

12. Atemgasschlauchanordnung nach einem der Ansprüche 1 bis 11, wobei die Heinzeinrichtung eine elektrische Widerstandsheizung ist.

13. Anschlusselement zum Verbinden einer Gebläseeinrichtung mit einer Atemgasschlauchanordnung, insbesondere nach einem der Ansprüche 1 bis 12, zur Zufuhr eines Atemgases zu einer Person, wobei die Atemgasschlauchanordnung eine Heizeinrichtung aufweist, und wobei das Anschlusselement an einem Ende einer Schlauchleitung (123) vorgesehen ist, um die Schlauchleitung mit einer Gebläseeinrichtung zu verbinden, und wobei das Anschlusselement als Anschlussstecker ausgebildet ist, der einen Atemgasdurchgangsquerschnitt sowie Kontaktelemente bereitstellt, über die die Heizeinrichtung mit Spannung versorgt wird.

14. CPAP-Vorrichtung, aufweisend:
eine Fördereinrichtung zur Förderung von Atemgas über eine flexible Atemgasschlauchanordnung, insbesondere nach einem der Ansprüche 1 bis 12, zu einem Patienten;
eine elektronische Regeleinrichtung zum Abstimmen des Förderdrückes der Fördereinrichtung; und
eine Anschlussstruktur, zum Verbinden mit einem Anschlusselement gemäß Anspruch 13, wobei die Anschlussstruktur eine Spannungsversorgung aufweist, um elektrische Spannung an die Kontaktelemente des Anschlusssteckers bereitzustellen, um Spannung an die Heizeinrichtung bereitzustellen, um das durch die Atemgasschlauchanordnung (123) geförderte Gas zu erwärmen.

## Claims

1. A flexible respiratory gas tube arrangement for supplying a respiratory gas to a person, comprising:
a flexible tube (123);
a flexible sheathing body (1);
a heating element for heating the gas supplied through the tube (123);
connector elements provided at the ends of the tube (123) for connecting the tube (123) with a breathing mask (112) or a blower means; wherein
the connector element is configured, for being connected with a blower means, as a connector plug comprising a respiratory gas passage cross-section (130) as well as contact elements (128) for supplying the heating element with power.

2. The respiratory gas tube arrangement according to claim 1, wherein the insulating sheathing body (1) can be detachably connected with the flexible tube (123).

3. The respiratory gas tube arrangement according to any one of claims 1 or 2, wherein the heating element is integrated in the sheathing body (1).

4. The respiratory gas tube arrangement according to any one of claims 1 to 3, wherein the sheathing body is multi-layered.

5. The respiratory gas tube arrangement according to claim 1, wherein the tube (123) is a heatable respiratory gas tube arrangement.

6. The respiratory gas tube arrangement according to claim 1, wherein the heating element is integrated in the tube (123).

7. The respiratory gas tube arrangement according to any one of claims 1 to 6, wherein the connector plug moreover comprises a pressure measuring tube connection 131.

8. The respiratory gas tube arrangement according to any one of claims 1 to 7, further comprising a respiratory gas humidifier (121).

9. The respiratory gas tube arrangement according to claim 8, wherein the respiratory gas humidifier (121) can be connected with a water source via a humidification line.

10. The respiratory gas tube arrangement according to claim 8 or 9, wherein the respiratory gas humidifier (121) is configured as a flexible tube piece disposed between the breathing mask (112) and the respiratory gas tube (123).

11. The respiratory gas tube arrangement according to any one of claims 1 to 10, wherein the flexible tube (123) is made of a spirally reinforced plastic material.

12. The respiratory gas tube arrangement according to any one of claims 1 to 11, wherein the heating element is an electrical resistance heating means.

13. A connector element for connecting a blower means with a respiratory gas tube arrangement, in particular according to any one of claims 1 to 12, for supplying a breathing gas to a person, wherein the respiratory gas tube arrangement comprises a heating element and wherein the connector element is provided at an end of a tube (123) for connecting the tube with a blower means, and wherein the connector element is configured as a connector plug providing a respiratory gas passage cross-section as well as contact elements for supplying the heating element with power.

14. A CPAP device comprising:
a supply means for supplying respiratory gas through a flexible respiratory gas tube
arrangement, in particular according to any one of claims 1 to 12, to a patient;
an electronic control means for adjusting the supply pressure of the supply means; and
a connector structure for connection with a connector element according to claim 13, wherein the connector structure comprises a power supply for supplying the contact elements of the connector plug with power in order to supply power to the heating means so as to heat the gas supplied through the respiratory gas tube arrangement (123).

## Revendications

1. Système de flexible souple pour acheminer un gaz respiratoire jusqu'à une personne, comprenant :
une conduite de flexible souple (123) ;
un corps de gaine flexible (1) ;
un dispositif de chauffage pour chauffer le gaz transporté à travers la conduite de flexible (123);
des éléments de raccordement prévus aux extrémités de la conduite de flexible (123), pour relier la conduite de flexible (123) à un masque respiratoire (112) et/ou à un dispositif de soufflerie ;
l'élément de raccordement destiné à la liaison avec un dispositif de soufflerie étant conçu comme fiche de raccordement, laquelle comporte une section de passage de gaz respiratoire (130) ainsi que des éléments de contact (128), par l'intermédiaire desquels le dispositif de chauffage est alimenté en tension.

2. Système de flexible de gaz respiratoire selon la revendication 1, le corps de gaine isolant (1) pouvant être relié de manière amovible à la conduite de flexible souple (123).

3. Système de flexible de gaz respiratoire selon l'une des revendications 1 ou 2, le dispositif de chauffage étant intégré dans le corps de gaine (1).

4. Système de flexible de gaz respiratoire selon l'une quelconque des revendications 1 à 3, le corps de gaine (1) étant réalisé avec plusieurs couches.

5. Système de flexible de gaz respiratoire selon la revendication 1, la conduite de flexible (123) étant un système de flexible de gaz respiratoire pouvant être chauffé.

6. Système de flexible de gaz respiratoire selon la revendication 1, le dispositif de chauffage étant intégré dans la conduite de flexible (123).

7. Système de flexible de gaz respiratoire selon l'une quelconque des revendications 1 à 6, la fiche de raccordement comportant en outre un raccordement de flexible de mesure de pression (131).

8. Système de flexible de gaz respiratoire selon l'une quelconque des revendications 1 à 7, avec en outre un humidificateur de gaz respiratoire (121).

9. Système de flexible de gaz respiratoire selon la revendication 8, l'humidificateur de gaz respiratoire (121) pouvant être relié à une source d'eau par l'intermédiaire d'une conduite d'humidification.

10. Système de flexible de gaz respiratoire selon la revendication 8 ou 9, l'humidificateur de gaz (121) étant conçu comme élément tubulaire flexible disposé entre le masque respiratoire (112) et le flexible de gaz respiratoire (123).

11. Système de flexible de gaz respiratoire selon l'une quelconque des revendications 1 à 10, la conduite de flexible souple (123) étant en matière plastique renforcée en spirale.

12. Système de flexible de gaz respiratoire selon l'une quelconque des revendications 1 à 11, le dispositif de chauffage étant un chauffage ohmique électrique.

13. Elément de raccordement destiné à relier un dispositif de soufflerie à un système de flexible, en particulier selon l'une quelconque des revendications 1 à 12, pour acheminer un gaz respiratoire jusqu'à une personne, le système de flexible de gaz respiratoire comportant un dispositif de chauffage, et l'élément de raccordement étant prévu à une extrémité d'une conduite de flexible (123) afin de relier ladite conduite de flexible à un dispositif de soufflerie, et l'élément de raccordement étant conçu comme fiche de raccordement qui fournit une section de passage de gaz respiratoire ainsi que des éléments de contact, par l'intermédiaire desquels le dispositif de chauffage est alimenté en tension.

14. Dispositif CPAP comprenant :
un dispositif d'alimentation destiné à acheminer du gaz respiratoire par l'intermédiaire d'un système de flexible, en particulier selon l'une quelconque des revendications 1 à 12, jusqu'à un patient;
un dispositif de régulation électronique pour adapter la pression d'alimentation du dispositif d'alimentation ; et
une structure de raccordement, destinée à la liaison avec un élément de raccordement selon la revendication 13, la structure de raccordement comportant une alimentation en courant pour fournir de la tension électrique aux éléments de contact de la fiche de raccordement, pour fournir de la tension au dispositif de chauffage, pour chauffer le gaz acheminé à travers le système de flexible (123).
